# EUROPEAN PATENT SPECIFICATION

(11) **EP 3 459 357 B1**
(45) Date of publication and mention of the grant of the patent: **22.01.2025**
(21) Application number: 17893394.1
(22) Date of filing: 20.01.2017
(51) Int. Cl.: A61K 38/27

(54) **METHOD FOR MANUFACTURING FEED CONTAINING SALMON GROWTH HORMONE, FEED CONTAINING SALMON GROWTH HORMONE, AND LIQUID CONTAINING SALMON GROWTH HORMONE**
VERFAHREN ZUR HERSTELLUNG VON LACHSWACHSTUMSHORMON-HALTIGEN FUTTERMITTELN, LACHSWACHSTUMSHORMON-HALTIGE FUTTERMITTEL UND LACHSWACHSTUMSHORMON-HALTIGE FLÜSSIGKEITEN
PROCÉDÉ DE FABRICATION D'ALIMENT CONTENANT UNE HORMONE DE CROISSANCE DE SAUMON, ALIMENT CONTENANT UNE HORMONE DE CROISSANCE DE SAUMON, ET LIQUIDE CONTENANT UNE HORMONE DE CROISSANCE DE SAUMON

(43) Date of publication of application: 27.03.2019
(73) Proprietor: Sanriku Fish Meal Co., Ltd., Kesennuma-shi, Miyagi 988-0033 (JP)
(72) Inventor: MORIYAMA, Shunsuke, Sagamihara-shi Kanagawa 252-0373 (JP); ASHIKAGA, Kieko, Kesennuma-shi Miyagi 988-0033 (JP); MORI, Kazuhiro, Kesennuma-shi Miyagi 988-0033 (JP)
(74) Representative: V.O.
(86) International application number: PCT/JP2017/001931
(87) International publication number: WO 2018/134968

(56) References cited:
- EP-A1- 2 008 523
- EP-A2- 0 157 423
- WO-A1-2014/030165
- JP-A- 2007 300 883
- JP-A- 2014 193 158
- JP-A- 2015 165 795
- JP-A- H04 141 050
- JP-A- H04 141 050
- HUAIXIA YIN ET AL: "Effects of Oil Extraction Methods on Physical and Chemical Properties of Red Salmon Oils ()", JOURNAL OF THE AMERICAN OIL CHEMISTS' SOCIETY, SPRINGER-VERLAG, BERLIN/HEIDELBERG, vol. 88, no. 10, 28 April 2011 (2011-04-28), pages 1641 - 1648, XP019947397, ISSN: 1558-9331, DOI: 10.1007/S11746-011-1824-X

## Description

### Technical Field

The present invention relates to a method for manufacturing a feed containing a salmon growth hormone, a feed containing a salmon growth hormone, and a liquid containing a salmon growth hormone.

### Background Art

It has been known that a growth hormone derived from fish such as salmon greatly promotes the growth of fish and shellfish. This fact is effective for aquaculture industry and the like of fish such as rainbow trout, eel, and Nishikigoi, or shellfish such as abalone, and attempts have been made to feed the fish and shellfish with a feed containing the growth hormone to accelerate the growth.

WO2014030165, EP0157423, JPH04141050, EP2008523, and Huaixia Yin et al. (Journal of the American Oil Chemists' Society, 88, No. 10, 28 April 2011 (2011-04-28), pages 1641-1648, XP019947397, ISSN: 1558-9331, DOI: 10.1007/S11746-011-1824-X) describe methods of obtaining certain compounds from fish products, and the like.

Patent Document 1 discloses a feed for marine invertebrates which contains a growth hormone derived from salmon, and particularly discloses that growth of the shellfish and the like can be easily and efficiently promoted in a short period of time.

### Citation List

### Patent Literature

[Patent Document 1] Japanese Unexamined Patent Application, First Publication No. 2005-95104

### Summary of Invention

### Technical Problem

In Patent Document 1, a salmon growth hormone is extracted at 4°C using a basic-buffer solution such as ammonium acetate (pH 9) in order to obtain the hormone. It is desirable to perform the operation at low temperature in order to prevent thermal denaturation of the salmon growth hormone and to maintain biological activity, but the manufacturing efficiency is poor because the activity is lost due to room or higher temperature storage of the salmon growth hormone extracted at low temperature, a solution containing the salmon growth hormone is freeze-dried for the storage, and the like.

In addition, in a method for manufacturing a feed, in general, frozen fish such as salmon is pulverized and boiled at 100°C. Moisture evaporates by the boiling and thus the manufacturing can be efficiently performed, but the growth hormone is thermally denatured, and therefore almost no hormone is left in the feed, and the biological activity of the growth hormone is lost.

The present invention has been made in view of the above circumstances, and provides an easy and efficient method for manufacturing a feed containing a salmon growth hormone with biological activity.

### Solution to Problem

That is, the present invention provides a method as defined in the claims.

### Advantageous Effects of Invention

According to the present invention, it is possible to provide the easy and efficient method for manufacturing a feed containing a salmon growth hormone with biological activity.

### Brief Description of Drawings

FIG. 1 is a schematic configuration diagram showing one embodiment of a method for manufacturing a feed containing a salmon growth hormone of the present invention.
FIG. 2 is a schematic configuration diagram showing another embodiment of the method for manufacturing a feed containing a salmon growth hormone of the present invention.
FIG. 3 is a schematic configuration diagram showing still another embodiment of the method for manufacturing a feed containing a salmon growth hormone of the present invention.
FIG. 4 is a graph showing a result of detecting the salmon growth hormone in a feed by HPLC in Example 1, the feed being heated at 50°C.
FIG. 5 is a graph showing a result of detecting the salmon growth hormone in the feed by HPLC in Example 2, the feed being heated at 60°C.
FIG. 6A is a graph showing a result of detecting the salmon growth hormone in a fluid component by HPLC in Example 3.
FIG. 6B is a graph showing a result of detecting the salmon growth hormone in a solid component by HPLC in Example 3.
FIG. 7A is a graph showing a result of detecting the salmon growth hormone in the fluid component by HPLC in Example 4.
FIG. 7B is a graph showing a result of detecting the salmon growth hormone in the solid component by HPLC in Example 4.
FIG. 8A is a graph showing a result of detecting a purified salmon growth hormone by HPLC in Test Example 1, the hormone being undergone a thermal tolerance test at 4°C.
FIG. 8B is a graph showing a result of detecting the purified salmon growth hormone by HPLC in Test Example 1, the hormone being undergone the thermal tolerance test at 50°C.
FIG. 8C is a graph showing a result of detecting the purified salmon growth hormone by HPLC in Test Example 1, the hormone being undergone the thermal tolerance test at 60°C.
FIG. 8D is a graph showing a result of detecting the purified salmon growth hormone by HPLC in Test Example 1, the hormone being undergone the thermal tolerance test at 70°C.
FIG. 9A is a graph showing a result of detecting a salmon growth hormone in a pituitary gland extract by HPLC in Test Example 1, the hormone being undergone the thermal tolerance test at 4°C.
FIG. 9B is a graph showing a result of detecting the salmon growth hormone in the pituitary gland extract by HPLC in Test Example 1, the hormone being undergone the thermal tolerance test at 50°C.
FIG. 9C is a graph showing a result of detecting the salmon growth hormone in the pituitary gland extract by HPLC in Test Example 1, the hormone being undergone the thermal tolerance test at 60°C.
FIG. 9D is a graph showing a result of detecting the salmon growth hormone in the pituitary gland extract by HPLC in Test Example 1, the hormone being undergone the thermal tolerance test at 70°C.
FIG. 10A is a graph showing a result of detecting a salmon growth hormone by HPLC in Test Example 2, the hormone being undergone a stability test at pH 7.0.
FIG. 10B is a graph showing a result of detecting the salmon growth hormone by HPLC in Test Example 2, the hormone being undergone the stability test at pH 9.0.
FIG. 10C is a graph showing a result of detecting the salmon growth hormone by HPLC in Test Example 2, the hormone being undergone the stability test at pH 11.0.
FIG. 11 is a graph showing results of detecting an expression level of IGF-I of liver cells derived from rainbow trout added with the salmon growth hormone by quantitative PCR in a quantitative test in Test Example 3.
FIG. 12 is a graph showing changes in weight of juvenile rainbow trout fed with the feed containing the salmon growth hormone in Test Example 4.

### Description of Embodiments

Hereinafter, embodiments of the present invention will be described in detail with reference to drawings as necessary. In the drawings, the same or corresponding parts are denoted by the same reference numerals, and a redundant description is omitted.

### <Method for Manufacturing Feed Containing Salmon Growth Hormone>

### (First Embodiment)

In one embodiment, the present invention provides a method for manufacturing a feed containing a salmon growth hormone, as defined in the claims.

According to the manufacturing method of the present embodiment, it is possible to manufacture the feed in which a content rate of the salmon growth hormone with biological activity is high, at high efficiency.

In the present specification, the term "salmon growth hormone" means growth hormone produced and released by salmon pituitary gland. A structure of the "salmon growth hormone" is well known in this field, and is described in, for example, Kawauchi et al., Arch. Biochem. BiopHys., 1986, 244:542-552, and the like.

The "salmon growth hormone" that can be used in the feed is not particularly limited, and examples of the hormone include growth hormone derived from the salmon pituitary gland, which is obtained by extracting the growth hormone produced and released by the salmon pituitary gland (containing rough extract, purified product, and the like), a recombinant salmon growth hormone manufactured by genetic recombination, and the like.

Large amounts of unused parts of salmon heads are being disposed during processing steps, and therefore, in the present invention, it is preferable to utilize growth hormone prepared with pituitary gland derived from the unused parts of the salmon heads. In addition, from the viewpoint of ease of extracting and purifying the salmon growth hormone, salmon with a low lipid content rate is preferable, and salmon with the lipid content rate of 0.1% or lower is more preferable.

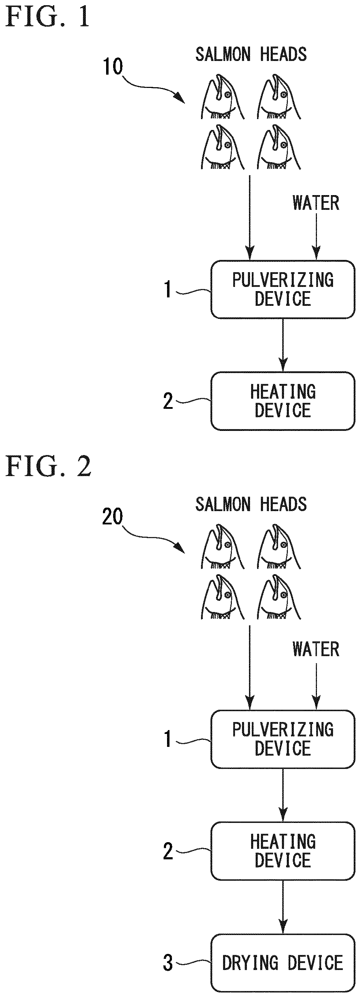

FIG. 1 is a schematic configuration diagram showing the first embodiment of the method for manufacturing the feed containing the salmon growth hormone of the present invention. Details of each step will be described below.

As a material, the large amounts of the unused parts of the salmon heads being disposed during the processing step described above, are collected, frozen-stored, and used. First, the salmon heads in the frozen state and water are added to a pulverizing device 1 so as to be pulverized and triturated. 1/4 to 1/3 volume of water with respect to a weight of the salmon heads is preferably added. In addition, the pulverizing device is not particularly limited, and examples of the device include a microcutter and the like.

Next, the pulverized salmon heads are added to a heating device 2 and heated, the salmon growth hormone is eluted and concentrated after evaporation of remaining moisture. In general, hormone is weak to heat and likely to be denatured by heat, but the inventors of the present invention have found that the salmon growth hormone has excellent thermal tolerance, and have discovered the present invention. Details of the reason thereof is unclear, but it is possible to presume that the salmon growth hormone is known to be a substance that is highly hydrophobic and is hardly soluble, and therefore has excellent thermal tolerance compared with other hormones.

The heating temperature is 50°C or higher and 90°C or lower, and more preferably 50°C or higher and 70°C or lower. By performing an elution step of the salmon growth hormone with the above-described lower limit or higher, it is possible to prevent thermal denaturation of the salmon growth hormone and to maintain the biological activity, and by eluting the salmon growth hormone, it is possible to improve the manufacturing efficiency. Meanwhile, by performing the elution step of the salmon growth hormone with the above-described upper limit or lower, the evaporation of the moisture becomes faster and thus the manufacturing can be performed efficiently, and furthermore, it is possible to prevent the thermal denaturation of the growth hormone and to maintain the biological activity of the growth hormone. A heating time can be appropriately adjusted according to the weight of the pulverized salmon heads, but is preferably 1 hour or longer and 2 hours or shorter, from the viewpoint of efficient manufacturing costs and energy costs.

In addition, the heating device is not particularly limited, and examples of the device include a tank capable of heating a material with hot water, steam, or the like, and retaining warmth.

### (Second Embodiment)

In addition, in one embodiment, the present invention further provides a method for manufacturing the feed containing the salmon growth hormone, as defined in the dependent claim.

According to the manufacturing method of the present embodiment, it is possible to remove the remaining moisture, thereby obtaining the feed containing the salmon growth hormone in a dry state.

FIG. 2 is a schematic configuration diagram showing another embodiment of the method for manufacturing the feed containing the salmon growth hormone of the present invention. Details of each step will be described below.

The same steps are carried out up to the pulverizing step and the heating step of the first embodiment. The salmon heads after the heating step is put into a drying device 3 so as to be dried. A drying temperature is preferably 50°C or higher and 70°C or lower, and a drying time can be appropriately adjusted according to the weight of the heated salmon heads, but is preferably 5 hours or longer and 12 hours or shorter.

In addition, the drying device is not particularly limited, and examples of the device include a low pressure dryer, a vacuum dryer, a dehumidifying dryer, a freeze dryer, a hot air dryer, and the like.

### (Third Embodiment)

In addition, in one embodiment, the present invention further provides a method for manufacturing the feed containing the salmon growth hormone, in accordance with the claims.

According to the manufacturing method of the present embodiment, the solid component and the fluid component can be used depending on a processing form of the feed. Furthermore, because the salmon growth hormone is being more eluted in the fluid component, a liquid with a high concentration of the salmon growth hormone can be obtained.

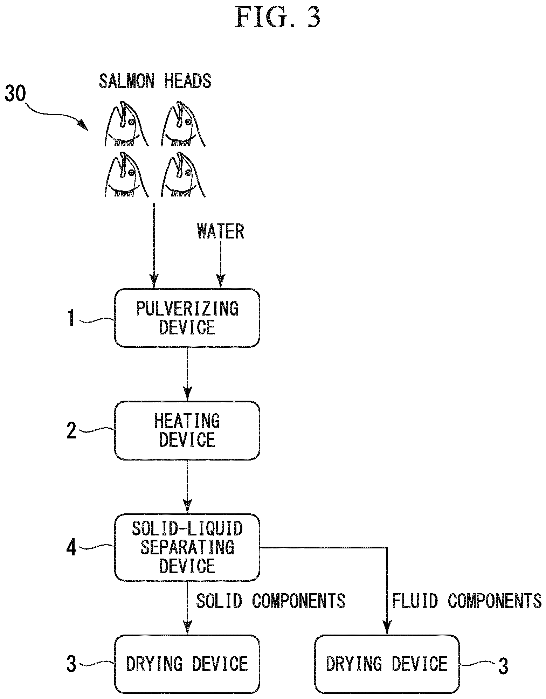

FIG. 3 is a schematic configuration diagram showing still another embodiment of the method for manufacturing the feed containing the salmon growth hormone of the present invention. Details of each step will be described below.

The same steps are carried out up to the pulverizing step and the heating step of the first embodiment. The salmon heads after the heating step is put into a solid-liquid separating device 4 so as to separate the solid component from the fluid component.

The solid-liquid separating device is not particularly limited, and examples of the device include a centrifugal separator capable of three-phase separation, and the like.

FIG. 3 shows a step of drying each of the solid component and the fluid component after the solid-liquid separating step by the drying device 3, but depending on use applications, the components can be used as they are without being dried.

### <Feed Containing Salmon Growth Hormone>

In one embodiment, the present invention provides the above-described manufacturing method according to the claims.

According to the method of the present embodiment, it is possible to easily promote the growth of fish and shellfish in a short period of time, and to improve productivity thereof.

The feed containing the salmon growth hormone of the present invention may be in any form of liquid and solid (gel and the like). In the case of the liquid form, the feed containing the salmon growth hormone can be dissolved in an appropriate solvent such as fresh water or seawater, and then used. The concentration of a solution in this case varies according to the fish and shellfish of a subject, a solid weight thereof, and the like, and thus may be appropriately determined. The concentration thereof is preferably 0.01 g/mL or more and 1 g/mL or less.

In addition, the feed containing the salmon growth hormone in the liquid form can be administered particularly to shellfish by a liquid bath or intramuscular injection. In a case of performing administration with the liquid bath, the feed containing the salmon growth hormone of a predetermined concentration is prepared, and a target shellfish is immersed in this solution for a predetermined time and frequency. Accordingly, the salmon growth hormone is incorporated into the body of the shellfish, and as a result, the growth thereof is promoted. The concentration of the salmon growth hormone used in the liquid bath is preferably 1 mg/L or more and 100 mg/L less for both cases of fish and abalone, for example.

Furthermore, in the case of the intramuscular injection, the feed containing the salmon growth hormone of a predetermined concentration is injected intramuscularly into a predetermined site of the shellfish. The concentration of the salmon growth hormone used in the intramuscular injection is preferably 10 ng/g body weight or more and 1000 ng/g body weight or less in the case of the fish, and is preferably 1 ng/g body weight or more and 100 ng/g body weight or less in the case of the abalone, for example. As the site on which the intramuscular injection is performed, adductor muscles are preferable.

The feed containing the salmon growth hormone in the liquid form is preferably adjusted to have pH of 7 to 9 in order to maintain the biological activity as a monomer. In order to adjust the pH, it is preferable to use, for example, sodium hydroxide, an ammonium acetate solution (pH 7 to 9), and the like.

In addition, the feed containing the salmon growth hormone in the solid form can be used as a feed for oral administration. Particularly for the shellfish, a gel form which particularly contains seaweed components and the like in addition to the salmon growth hormone and in which the salmon growth hormone is retained within the gel, is preferable among solid forms.

As a method for administering the feed for oral administration, the fish and shellfish are fed for a predetermined period and number of times under the same environments as those of the fish and shellfish. The concentration of the salmon growth hormone in the feed containing the salmon growth hormone varies according to the fish and shellfish of the subject, the solid weight thereof, and the like, and thus may be appropriately determined. For example, in the case of the abalone (body weight of about 2 g to 3 g, shell length of about 3 to 5 cm), it is preferable that a concentration range is within 0.5 mg/8 g to 5 mg/8 g. By setting the concentration range as above, the growth of the abalone can be efficiently promoted in a short period of time. An interval and a frequency of administration may be determined according to the purpose and situation and are, for example, 20 times to 60 times every 7 days to 14 days. Furthermore, for example, in the case of the fish, it is preferable that the concentration range is within 100 ng/g to 5000 ng/g.

The feed containing the salmon growth hormone in the solid form can be incorporated into the body of the fish and shellfish without affecting the fish and shellfish by handling stress while suppressing deactivation of the salmon growth hormone.

The feed containing the salmon growth hormone made by the method of the present invention may contain feed additives generally used as feed components, and examples of the components include antibiotics, vitamins, minerals, and the like.

In the present invention, types of the fish and shellfish are not particularly limited, and examples thereof include fish that are cultivated actively in aquaculture industry, such as ayu, carp, and trout, shellfish such as abalone, turban shell, barnacle, oyster, and scallop, crustaceans such as shrimp and crab, sea urchins, and the like. However, the examples are not limited thereto, and for example, the present invention can also be applied to invertebrates such as pond snail and Marsh periwinkles that inhabit fresh water. Oysters and scallops are suitable for the liquid bath.

### <Liquid Containing Salmon Growth Hormone>

In one embodiment, the present invention provides a method according to the claims.

According to the liquid containing the salmon growth hormone of the present embodiment, it is possible to easily promote the growth of the fish and shellfish in a short period of time, and to improve the productivity thereof. In addition, the liquid containing the salmon growth hormone has a high concentration of the salmon growth hormone, and thus can be used as it is as a liquid feed, and furthermore, the liquid can be mixed with other nutritional ingredients and the like and subjected to a freely customized processing.

A method for manufacturing the liquid containing the salmon growth hormone of the present embodiment is the same as that of the third embodiment of the above-described methods for manufacturing the feed containing the salmon growth hormone. The salmon heads after the pulverizing step and the heating step can be separated by the solid-liquid separating device so as to obtain the fluid component. The fluid component after the solid-liquid separating step may be used as they are, but collagen is dissolved in the fluid component. Accordingly, by freezing and re-thawing the fluid component, collagen is gelatinized and precipitates, and the salmon growth hormone in the fluid can be easily purified.

### Examples

### [Example 1] Manufacturing of Feed Containing Salmon Growth Hormone (Heating at 50°C)

### (1) Pulverizing Step

Water of a total of 180 kg was added to frozen-stored salmon heads (600 kg), and then the mixture was pulverized and triturated with a microcutter (to 1.5 mm).

### (2) Heating Step

The pulverized salmon heads were placed in a warming tank and stirred for 30 minutes for thawing, and then warmed while heating for about 30 minutes after reaching 50°C.

### (3) Drying Step

The obtained fluid component and solid component were dried with a vacuum dryer set at 70°C.

### (4) Detection of Salmon Growth Hormone

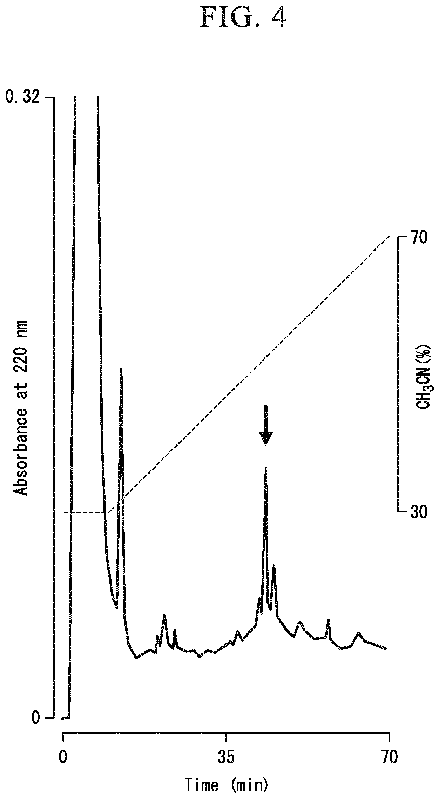

350 ml of distilled water was added to the obtained dry solid matter and the mixture was stirred at 4°C for 24 hours. The suspension was centrifuged (6,000 rpm, 30 minutes, 4°C), and then the supernatant was collected. The obtained supernatant was centrifuged again (145,000 rpm, 10 minutes, 4°C), and then the supernatant was added to high performance liquid chromatography (HPLC) (manufactured by JASCO Corporation) to calculate the elution position and the amount of the salmon growth hormone. The flow rate was 1 ml/min and the column temperature was 40°C. The results of HPLC are shown in FIG. 4. The concentration of the salmon growth hormone in the feed containing the salmon growth hormone was 77.1 µg/g (77.1 mg/kg). When converting into a conventional feed containing the salmon growth hormone (concentration of the salmon growth hormone is 2 µg/g), the amount contained of the salmon growth hormone was equivalent to 38.55 kg of the feed.

### [Example 2] Manufacturing of Feed Containing Salmon Growth Hormone (Heating at 60°C)

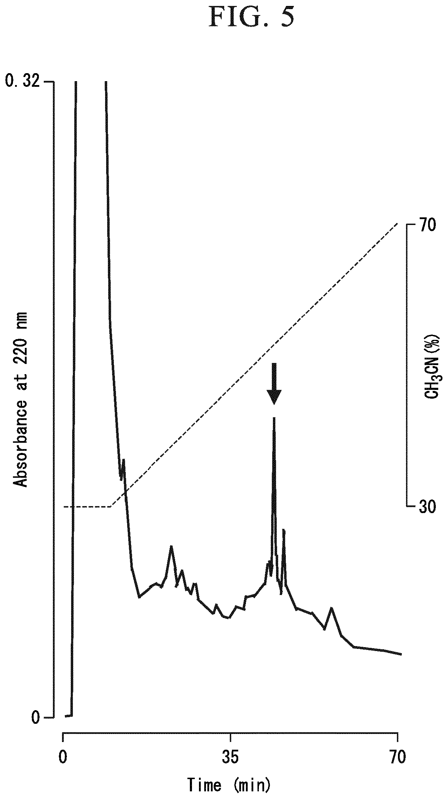

The feed containing the salmon growth hormone was manufactured and the elution position and the amount of the salmon growth hormone in the feed were calculated in the same method as in Example 1 except that the temperature in the heating step was changed to 60°C. The results of HPLC are shown in FIG. 5. The concentration of the salmon growth hormone in the feed containing the salmon growth hormone was 85.2 µg/g (85.2 mg/kg). When converting into a conventional feed containing the salmon growth hormone (concentration of the salmon growth hormone is 2 µg/g), the amount contained of the salmon growth hormone was equivalent to 42.60 kg of the feed.

### [Example 3] Manufacturing of Feed Containing Salmon Growth Hormone (Heating at 50°C and Solid Liquid Separation)

### (1) Pulverizing Step

The pulverizing step was carried out in the same method as (1) of Example 1.

### (2) Heating Step

The heating step was carried out in the same method as (2) of Example 1.

### (3) Solid-Liquid Separating Step

The heated salmon heads were separated into the fluid component and the solid component by a three-phase separator.

### (4) Drying Step

The obtained fluid component and solid component were dried with the vacuum dryer set at 50°C, for 7 hours and 30 minutes for the solid component, and for 11 hours and 15 minutes for the fluid component.

### (5) Detection of Salmon Growth Hormone

### (i) Extraction of Salmon Growth Hormone

The solid matter (0.5 g) after drying with the vacuum dryer was added to 2 mL of 0.2 M ammonium acetate (pH 9) and triturated with POLYTRON homogenizer (manufactured by Polytron Co.). Thereafter, 2 mL of 0.2 M ammonium acetate (pH 9) was added to the triturated product, and the mixture was extracted at 4°C for 1 hour. After centrifugation, 3 mL of a supernatant containing the salmon growth hormone was obtained.

### (ii) Concentration of Salmon Growth Hormone

Next, to concentrate the salmon growth hormone, 1 mL of the supernatant was added to Sep-Pak C18 cartridge (manufactured by Waters) equilibrated with 0.1% trifluoroacetic acid (TFA) (9.1%). After washing with 5 mL of 0.1% TFA, 4 mL of 20% acetonitrile (containing 0.1% TFA) and then 4 mL of 60% acetonitrile (containing 0.1% TFA) were added thereto, and proteins adsorbed on the cartridge was eluted, followed by freeze-drying.

### (iii) Detection with HPLC

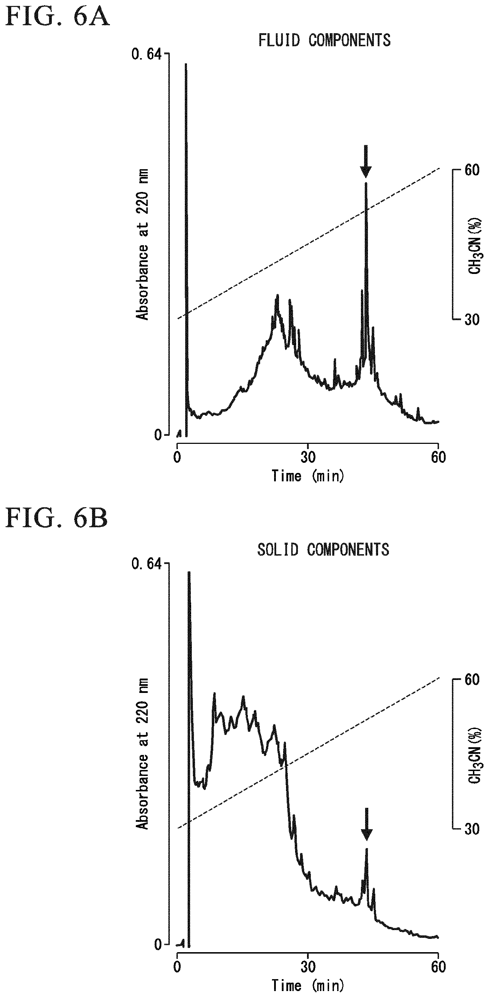

0.6 g of the freeze-dried solid content was dissolved in 500 µL of 0.1% TFA and an ODS-120T column (manufactured by Tosoh Corporation) was added to high performance liquid chromatography (HPLC) (manufactured by JASCO Corporation). The elution position and the content of the salmon growth hormone were calculated by a linear concentration gradient method for raising the concentration from 30% to 60% in 60 minutes using acetonitrile containing 0.1% TFA. The flow rate was 1 mL/min and the column temperature was 40°C. The results of HPLC are shown in FIG. 6A and FIG. 6B. The concentration of the salmon growth hormone in the dry matter derived from the liquid component containing the salmon growth hormone was 15 µg/g, and the concentration of the salmon growth hormone in the dry matter derived from the solid component the containing a salmon growth hormone was 3 µg/g.

### [Example 4] Manufacturing of Feed Containing Salmon Growth Hormone (Heating at 70°C and Solid Liquid Separation)

### (1) Pulverizing Step

The pulverizing step was carried out in the same method as (1) of Example 1.

### (2) Heating Step

The heating step was carried out in the same method as (2) of Example 1.

### (3) Solid-Liquid Separating Step

The heated salmon heads were separated into the fluid component and the solid component by the three-phase separator.

### (4) Drying Step

The obtained fluid component and solid component were dried with the vacuum dryer set at 70°C, for 5 hours for the solid component, and for 10 hours and 15 minutes for the fluid component.

### (5) Detection of Salmon Growth Hormone

### (i) Extraction of Salmon Growth Hormone

The heating step was carried out in the same method as (i) of (5) of Example 3.

### (ii) Concentration of Salmon Growth Hormone

The heating step was carried out in the same method as (ii) of (5) of Example 3.

### (iii) Detection with HPLC

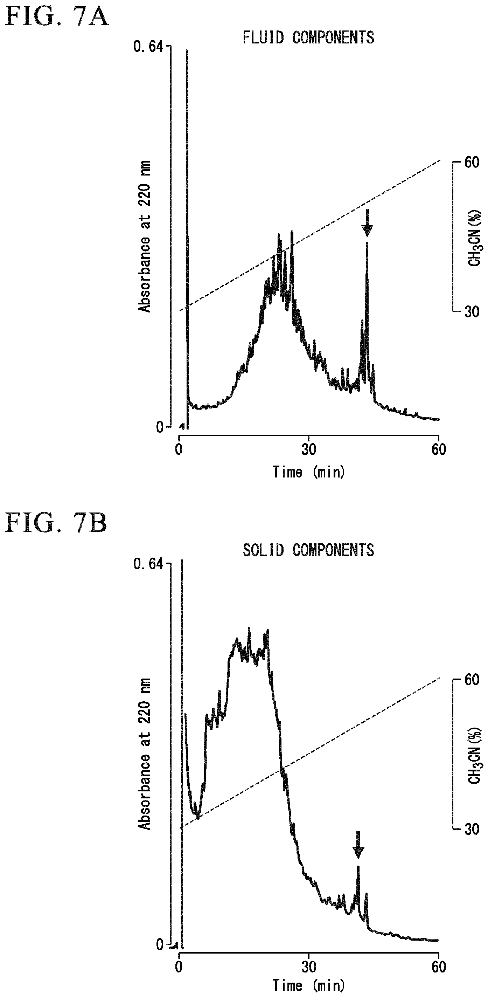

The heating step was carried out in the same method as (iii) of (5) of Example 3. The results of HPLC are shown in FIG. 7A and FIG. 7B. The concentration of the salmon growth hormone in the dry matter derived from the liquid component containing the salmon growth hormone was 11 µg/g, and the concentration of the salmon growth hormone in the dry matter derived from the solid component containing the salmon growth hormone was 1.5 µg/g.

### [Test Example 1] Thermal Tolerance Test of Salmon Growth Hormone

### (1) Preparation of Sample

Purified growth hormones derived from the salmon pituitary gland were placed in 0.05 M ammonium acetate (pH 9.0) and kept for 10 minutes at 4°C, 50°C, 60°C, and 70°C, respectively (these were taken as purified salmon growth hormones).

In addition, substances in the salmon brain ventricle were put in 400 µL of 0.2 M ammonium acetate solution (pH 9.0), and kept for 10 minutes at 4°C, 50°C, 60°C, and 70°C, respectively (these were taken as salmon growth hormones in pituitary gland extract). In regard to the salmon growth hormone in the pituitary gland extract, after lapse of 10 minutes, the substances in the brain ventricle were triturated using a pestle and the salmon growth hormone was extracted for 10 minutes at 4°C. The extract was centrifuged and the supernatant (350 µL) was collected.

### (2) Detection of Salmon Growth Hormone

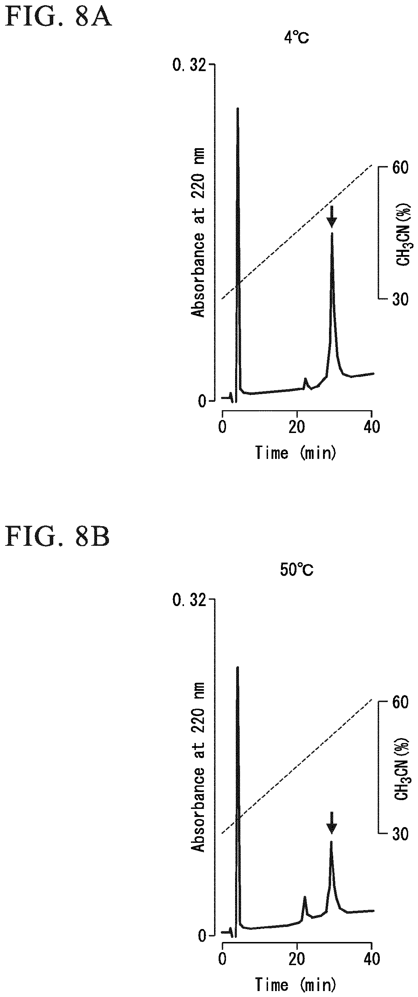

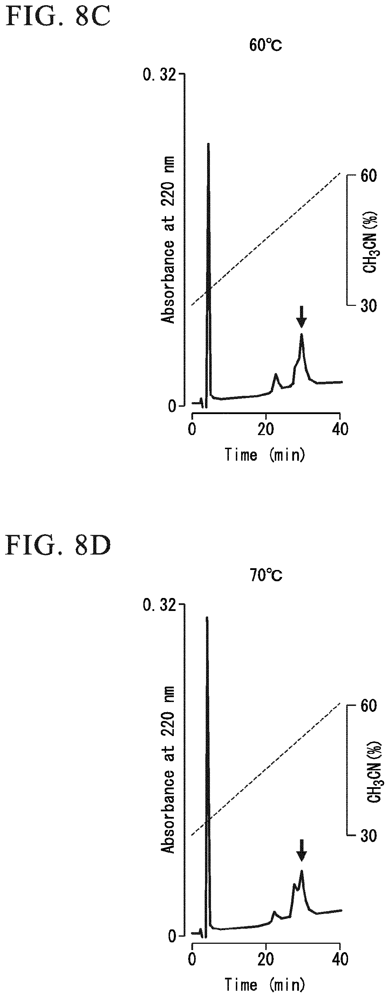

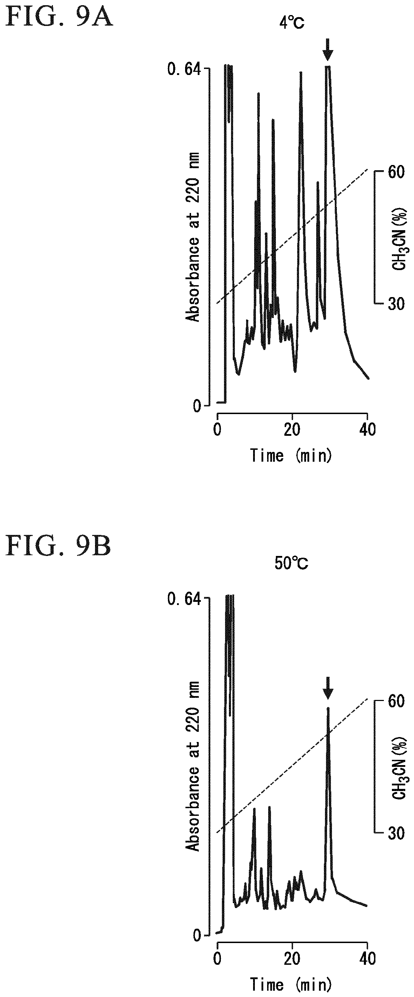

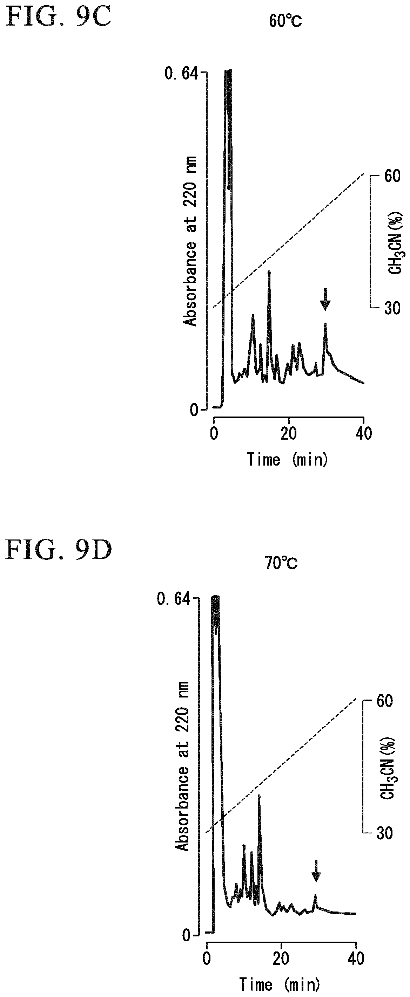

500 µL of 0.1% TFA was added to each sample (350 µL) of the purified salmon growth hormone and the salmon growth hormone in the pituitary gland extract, the sample being treated under the temperature conditions at 4°C, 50°C, 60°C, and 70°C, and the mixtures were diluted. Among the samples, 500 µL was added to HPLC, and the elution position and the content of the salmon growth hormone were verified. The HPLC results of the purified growth hormone are shown in FIGS. 8A to 8D. In addition, the results of HPLC of the salmon growth hormone in the pituitary gland extract are shown in FIGS. 9A to 9D.

In both the purified growth hormone and the salmon growth hormone in the pituitary gland extract, the content of the growth hormone in the sample decreased as the temperature increased with respect to the content of the growth hormone in the sample treated under the condition of the temperature at 4°C, but it became clear that the salmon growth hormone is still left in the sample treated at 70°C. The salmon growth hormone is known to be highly hydrophobic, and therefore can be presumed to have a high degree of heat resistance compared with other hormones.

### [Test Example 2] pH Stability Test of Salmon Growth Hormone

### (1) Preparation of Sample

Solutions in which the purified salmon growth hormone derived from salmon pituitary gland was dissolved were prepared to have pH 7.0, pH 9.0, and pH 11.0 using a pH adjuster, and kept at room temperature for 60 minutes.

### (2) Detection of Salmon Growth Hormone

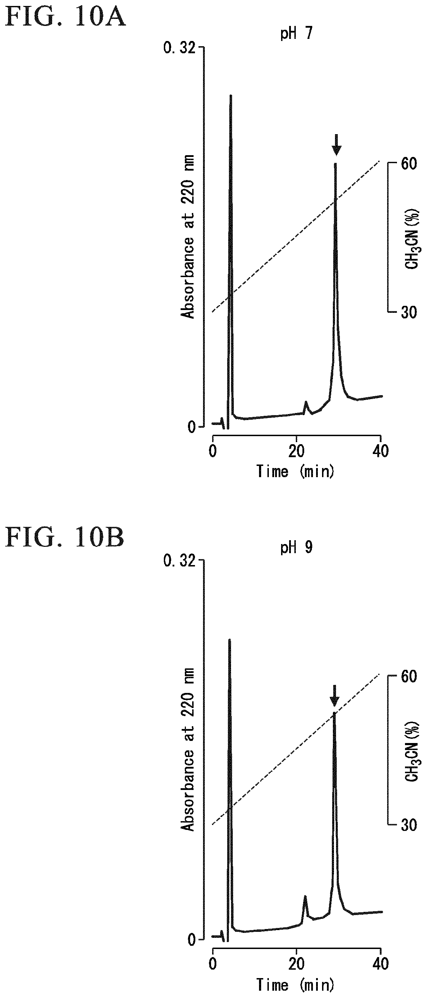

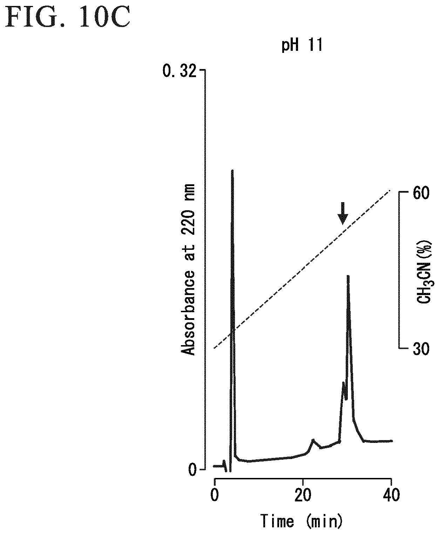

The detection was carried out in the same method as (2) of Test Example 1. The results are shown in FIGS. 10A to 10C.

As pH became basic, the amount detected of the salmon growth hormone decreased, but it became clear that a sufficient amount of the salmon growth hormone can be obtained even at pH 11. Based on this finding, it can be presumed that the salmon growth hormone is highly resistant to base.

### [Test Example 3] Quantitative Test of Expression Level of IGF-I of Liver Cells Derived from Rainbow Trout Added with Salmon Growth Hormone

### (1) Preparation of Sample

Each of the feed containing the salmon growth hormone obtained in Example 1 and Example 2, and the purified salmon growth hormone as a control was added to Minimum Essential Medium Eagle (Sigma-Aldrich) so that the amount of the each salmon growth hormone became 0, 10, 100, and 1000 ng/mL. 0.5 mL of Minimum Essential Medium Eagle (Sigma Aldrich) containing the salmon growth hormone of the above-described concentration were respectively added to liver cells derived from rainbow trout, which had been seeded and cultured in advance, and the mediums were cultured at 10°C for 24 hours.

### (2) Quantification of Expression Level of IGF-I of Liver Cells Derived from Rainbow Trout by Quantitative PCR

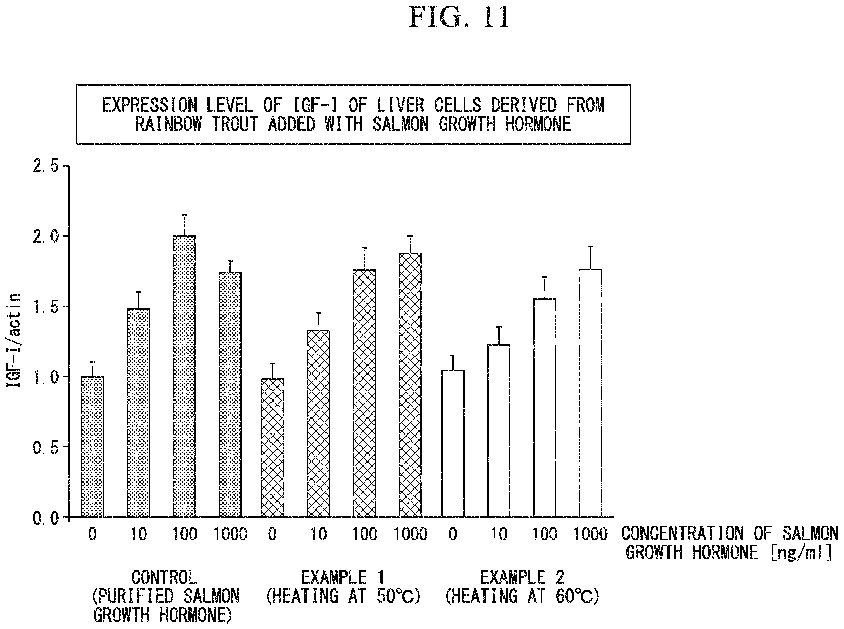

The cultured cells were collected, mRNA was extracted, and the expression level of IGF-I (Insulin like growth factors) was quantitatively determined by quantitative PCR. The results are shown in FIG. 11. FIG. 11 shows a relative ratio in each of the feeds containing the salmon growth hormone obtained in Example 1 and Example 2 and the purified salmon growth hormone as the control when the expression level of IGF-I in a culture solution not containing the salmon growth hormone was taken as 1.

IGF-I is a single chain polypeptide consisting of 70 amino acids and is one of factors mediating the growth promoting action of growth hormone (GH) in bone and somatic cells. Secretion of IGF-I depends on the growth hormone (GH) and is produced in various organs. Therefore, the expression level of IGF-I can be used as an index of a growth effect by the salmon growth hormone.

In FIG. 11, the expression level of IGF-I increased in a concentration-dependent manner in the feed containing the salmon growth hormone obtained in Example 1 and Example 2 and the purified salmon growth hormone. Therefore, it became clear that the salmon growth hormone in the feed obtained by the heat treatment at 50°C and 60°C in Example 1 and Example 2 had a sufficient degree of the biological activity.

### [Test Example 4] Evaluation on Growth of Juvenile Rainbow Trout Fed with Feed Containing Salmon Growth Hormone

### (1) Preparation of Feed for Juvenile Rainbow Trout

Using the growth hormone solution prepared from the feed containing the salmon growth hormone obtained in Example 1 and Example 2, a feed for juvenile rainbow trout was prepared so that the content of the salmon growth hormone became 2.5 µg/g for each feed. As a control, a feed not containing the salmon growth hormone was also prepared.

### (2) Breeding of Juvenile Rainbow Trout

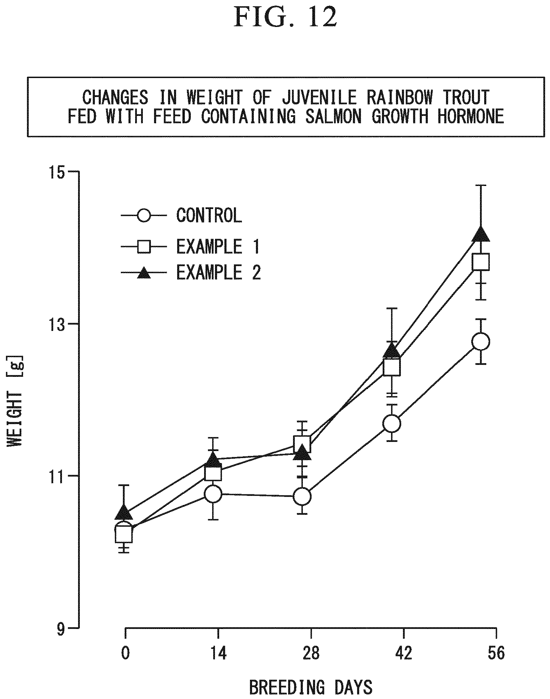

The juvenile rainbow trouts (n = 15 tail) were fed with the feed by 1% amount of whole body weight of the trout twice a day, and were bred for 56 days. The weight of the juvenile fish was measured every 14 days, and the results are shown in FIG. 12.

In the juvenile fish fed with the feed containing the salmon growth hormone obtained in Example 1 and Example 2, a significant difference in an increase amount of the weight was recognized from 14 days after breeding, compared to the juvenile fish fed with the control feed. In addition, after 56 days, in the juvenile fish fed with the feed containing the salmon growth hormone obtained in Example 1 and Example 2, the weight increased by about 1.5 g, which is a large increase, compared to the juvenile fish fed with the control feed. Therefore, it became clear that the feed containing the salmon growth hormone obtained in Example 1 and Example 2 had the effect of promoting the growth of the juvenile rainbow trouts.

Based on the above-described results, it became clear that, according to the present invention, it is possible to manufacture the feed in which the content rate of the salmon growth hormone with the biological activity is high, at high efficiency. In addition, it became clear that, according to the present disclosure it is possible to easily promote the growth of the fish and shellfish in a short period of time, and to improve the productivity thereof.

### Industrial Applicability

According to the present invention, it is possible to manufacture the feed in which the content rate of the salmon growth hormone with the biological activity is high, at high efficiency. In addition, according to the present disclosure it is possible to easily promote the growth of the fish and shellfish in a short period of time, and to improve the productivity thereof.

### Reference Signs List

1: pulverizing device, 2: heating device, 3: drying device, 4: solid-liquid separating device, 10, 20, 30: manufacturing device of feed containing salmon growth hormone

## Claims

1. A method for manufacturing a liquid containing a salmon growth hormone, comprising:
a heating step of heating a pulverized salmon head at 50°C or higher and 90°C or lower,
a separating step of separating a solid component from a fluid component of the heated salmon head, and
a freezing and re-thawing step of freezing and re-thawing the fluid component.

2. The method for manufacturing a liquid containing a salmon growth hormone according to claim 1, further comprising:
a drying step of drying the fluid component.

## Patentansprüche

1. Verfahren zum Herstellen einer Flüssigkeit, enthaltend ein Wachstumshormon für Lachse, umfassend:
einen Erhitzungsschritt zum Erhitzen eines pulverisierten Lachskopfes auf 50 °C oder höher und 90 °C oder niedriger,
einen Trennschritt zum Trennen einer festen Komponente von einer flüssigen Komponente des erhitzten Lachskopfes und
einen Gefrier- und Wiederauftauschritt zum Gefrieren und Wiederauftauen der flüssigen Komponente.

2. Verfahren zum Herstellen einer Flüssigkeit, enthaltend ein Wachstumshormon für Lachse, nach Anspruch 1, ferner umfassend:
einen Trocknungsschritt zum Trocknen der flüssigen Komponente.

## Revendications

1. Procédé pour fabriquer un liquide contenant une hormone de croissance de saumon, comprenant :
une étape de chauffage dans laquelle une tête de saumon pulvérisée est chauffée à 50 °C ou plus et 90 °C ou moins,
une étape de séparation dans laquelle un composant solide est séparé d'un composant fluide de la tête de saumon chauffée, et
une étape de congélation et de décongélation dans laquelle le composant fluide est congelé et décongelé.

2. Procédé pour fabriquer un liquide contenant une hormone de croissance de saumon selon la revendication 1, comprenant en outre :
une étape de séchage dans laquelle le composant fluide est séché.
